# EUROPEAN PATENT APPLICATION

(11) **EP 1 990 056 A1**
(43) Date of publication of application: **12.11.2008**
(21) Application number: 07737440.3
(22) Date of filing: 27.02.2007
(51) Int. Cl.: A61K 38/00, A61P 1/02, A61P 43/00, C12N 15/09

(54) **TOOTH ROOT FORMATION PROMOTER AND METHOD FOR PROMOTION OF TOOTH ROOT FORMATION**

(30) Priority: 28.02.2006 JP 2006053842
(71) Applicant: National University Corporation Tokyo Medical and Dental University, Tokyo 113-8510 (JP)
(72) Inventor: OTA, Masato, Chiba-shi, Chiba 262-0033 (JP); KAWASHIMA, Nobuyuki, Tokyo 157-0066 (JP); NAKAHARA, Taka, Tokyo 102-0074 (JP); ISEKI, Sachiko, Tokyo 112-0002 (JP)
(74) Representative: Graf von Stosch, Andreas
(86) International application number: PCT/JP2007/053638
(87) International publication number: WO 2007/099953

(57) **Abstract**

The present invention provides a tooth root formation promoting factor and a method for promotion of tooth root formation, which can promote tooth root formation and which are useful in various aspects of dental therapy. Specifically, the tooth root formation promoting factor contains, as an active ingredient, proteins belonging to the FGF8 subfamily (i.e., fibroblast growth factors); and the method includes administering proteins belonging to the FGF8 subfamily to a mammalian.

SEQUENCE LISTING

<110> National University Corporation Tokyo Medical and Dental University

<120> Tooth Root Formation Promoting Factors And Method For Promotion Of Tooth Root
Formation

<130> N-MD001-07P

<150> JP 2006-053842
<151> 2006-02-28

<160> 6

<170> PatentIn version 3.1

<210> 1
<211> 1546
<212> DNA
<213> human

<220>
<221> CDS
<222> (538)..(1161)
<223>

<300>
<308> AF075292

<309> 1998-10-02

<400> 1

cacggccgga gagacgcgga ggaggagaca tgagccggcg ggcgcccaga cggagcggcc 60

gtgacgcttt cgcgctgcag ccgcgcgccc cgaccccgga gcgctgaccc ctggccccac 120

gcagctccgc gcccgggccg gagagcgcaa ctcggcttcc agacccgccg cgcatgctgt 180

ccccggactg agccgggcag ccagcctccc acggacgccc ggacggccgg ccggccagca 240

gtgagcgagc ttccccgcac cggccaggcg cctcctgcac agcggctgcc gccccgcagc 300

ccctgcgcca gcccggaggg cgcagcgctc gggaggagcc gcgcggggcg ctgatgccgc 360

agggcgcgcc gcggagcgcc ccggagcagc agagtctgca gcagcagcag ccggcgagga 420

gggagcagca gcagcggcgg cggcggcggc ggcggcggcg gaggcgcccg gtcccggccg 480

cgcggagcgg acatgtgcag gctgggctag gagccgccgc ctccctcccg cccagcg 537

atg tat tca gcg ccc tcc gcc tgc act tgc ctg tgt tta cac ttc ctg 585
Met Tyr Ser Ala Pro Ser Ala Cys Thr Cys Leu Cys Leu His Phe Leu
1               5                   10                  15

ctg ctg tgc ttc cag gta cag gtg ctg gtt gcc gag gag aac gtg gac 633
Leu Leu Cys Phe Gln Val Gln Val Leu Val Ala Glu Glu Asn Val Asp
            20                  25                  30

ttc cgc atc cac gtg gag aac cag acg cgg gct cgg gac gat gtg agc 681
Phe Arg Ile His Val Glu Asn Gln Thr Arg Ala Arg Asp Asp Val Ser
        35                  40                  45

cgt aag cag ctg cgg ctg tac cag ctc tac agc cgg acc agt ggg aaa 729
Arg Lys Gln Leu Arg Leu Tyr Gln Leu Tyr Ser Arg Thr Ser Gly Lys
    50                  55                  60

cac atc cag gtc ctg ggc cgc agg atc agt gcc cgc ggc gag gat ggg 777
His Ile Gln Val Leu Gly Arg Arg Ile Ser Ala Arg Gly Glu Asp Gly
65                  70                  75                  80

gac aag tat gcc cag ctc cta gtg gag aca gac acc ttc ggt agt caa 825
Asp Lys Tyr Ala Gln Leu Leu Val Glu Thr Asp Thr Phe Gly Ser Gln
                85                  90                  95

gtc cgg atc aag ggc aag gag acg gaa ttc tac ctg tgc atg aac cgc 873
Val Arg Ile Lys Gly Lys Glu Thr Glu Phe Tyr Leu Cys Met Asn Arg
            100                 105                 110

aaa ggc aag ctc gtg ggg aag ccc gat ggc acc agc aag gag tgt gtg 921
Lys Gly Lys Leu Val Gly Lys Pro Asp Gly Thr Ser Lys Glu Cys Val
        115                 120                 125

ttc atc gag aag gtt ctg gag aac aac tac acg gcc ctg atg tcg gct 969
Phe Ile Glu Lys Val Leu Glu Asn Asn Tyr Thr Ala Leu Met Ser Ala
    130                 135                 140

aag tac tcc ggc tgg tac gtg ggc ttc acc aag aag ggg cgg ccg cgg 1017
Lys Tyr Ser Gly Trp Tyr Val Gly Phe Thr Lys Lys Gly Arg Pro Arg
145                 150                 155                 160

aag ggc ccc aag acc cgg gag aac cag cag gac gtg cat ttc atg aag 1065
Lys Gly Pro Lys Thr Arg Glu Asn Gln Gln Asp Val His Phe Met Lys
                165                 170                 175

cgc tac ccc aag ggg cag ccg gag ctt cag aag ccc ttc aag tac acg 1113
Arg Tyr Pro Lys Gly Gln Pro Glu Leu Gln Lys Pro Phe Lys Tyr Thr
            180                 185                 190

acg gtg acc aag agg tcc cgt cgg atc cgg ccc aca cac cct gcc tag 1161
Thr Val Thr Lys Arg Ser Arg Arg Ile Arg Pro Thr His Pro Ala
        195                 200                 205

gccaccccgc cgcggcccct caggtcgccc tggccacact cacactccca gaaaactgca 1221

tcagaggaat atttttacat gaaaaataag gaagaagctc tatttttgta cattgtgttt 1281

aaaagaagac aaaaactgaa ccaaaactct tggggggagg ggtgataagg attttattgt 1341

tgacttgaaa cccccgatga caaaagactc acgcaaaggg actgtagtca acccacaggt 1401

gcttgtctct ctctaggaac agacaactct aaactcgtcc ccagaggagg acttgaatga 1461

ggaaaccaac actttgagaa accaaagtcc tttttcccaa aggttctgaa aggaaaaaaa 1521

aaaaaaaaca aaaaaaaaaa aaaaa 1546

<210> 2

<211> 207

<212> PRT

<213> human

<400> 2

Met Tyr Ser Ala Pro Ser Ala Cys Thr Cys Leu Cys Leu His Phe Leu

1               5                   10                  15

Leu Leu Cys Phe Gln Val Gln Val Leu Val Ala Glu Glu Asn Val Asp

            20                  25                  30

Phe Arg Ile His Val Glu Asn Gln Thr Arg Ala Arg Asp Asp Val Ser

        35                  40                  45

Arg Lys Gln Leu Arg Leu Tyr Gln Leu Tyr Ser Arg Thr Ser Gly Lys

    50                  55                  60

His Ile Gln Val Leu Gly Arg Arg Ile Ser Ala Arg Gly Glu Asp Gly

65                  70                  75                  80

Asp Lys Tyr Ala Gln Leu Leu Val Glu Thr Asp Thr Phe Gly Ser Gln
                85                  90                  95

Val Arg Ile Lys Gly Lys Glu Thr Glu Phe Tyr Leu Cys Met Asn Arg
            100                 105                 110

Lys Gly Lys Leu Val Gly Lys Pro Asp Gly Thr Ser Lys Glu Cys Val
        115                 120                 125

Phe Ile Glu Lys Val Leu Glu Asn Asn Tyr Thr Ala Leu Met Ser Ala
    130                 135                 140

Lys Tyr Ser Gly Trp Tyr Val Gly Phe Thr Lys Lys Gly Arg Pro Arg
145                 150                 155                 160

Lys Gly Pro Lys Thr Arg Glu Asn Gln Gln Asp Val His Phe Met Lys
                165                 170                 175

Arg Tyr Pro Lys Gly Gln Pro Glu Leu Gln Lys Pro Phe Lys Tyr Thr

            180                 185                 190

Thr Val Thr Lys Arg Ser Arg Arg Ile Arg Pro Thr His Pro Ala
        195                 200                 205

<210> 3
<211> 9714
<212> DNA
<213> human

<220>
<221> CDS
<222> (2091)..(2122)
<223>

<220>
<221> CDS
<222> (2215)..(2251)
<223>

<220>
<221> CDS
<222> (2775)..(2861)
<223>

<220>

<221>   CDS

<222>   (3079)..(3259)

<223>

<220>

<221>   CDS

<222>   (6422)..(6528)

<223>

<220>

<221>   CDS

<222>   (7372)..(7659)

<223>

<300>

<308>   AF520763

<309>   2002-06-20

<400>   3
acttgtgtcc acctgacttg tctccgcctg atggacccac ctccccatgg gctttagagg        60
agaccagtgc ctgccaagcc tgtggcagat acaagtgctc actaactggt ggtgttcctg       120
gtgataatct gttgggagga ggtgaaggca ggtcttcctg gggaagttgc aaataggctg       180
aagtctacag gctcagccac ttcccccatc attcattcat tcattcattc aacgaactct       240
taattgcaga acagctctgc ccagccttgt gccacgagct gtgatttctg agctcataag       300
acatggtcct tgtgggcaag gtctgtgtgg gcaagagccc actgaacaga ctcctggagg 360
aggatggcaa gtgtgctctg gaagtctact tctctgagga gggataagat cttggaacgc 420
accctctaac tgttttccca tctctccatt gctcagtgtt tgcatttatg gctgggagtt 480
ggaaagatgg ggcacaggga aaggtgggac acagagcagg gagggaggca gggcctggct 540
gtgctgctgg actagggctg ccgctaattt tggcctaatg gcttcctggg accacaccaa 600
accctccagc cctaccccca ggggaatgga gggtcactgg ggaaggggag gttgtaagtg 660
aagacagcag tctggtaggg catcacctgc tcctcacaag gcacctgtcc cagagcatct 720
ctgtccaccc aggctgctcc cacagaagca gcatgggggc tcagggtttc cagaaacctc 780
aggggggaag ccacaaggtg gggggctagg aaaggcttag acaagtgcct accttctcct 840
atgccccaag cccagctgac cagcccctgc ctagaataca gtaggtgtgt aaatacctgt 900
gaaatgaaaa acgagtgggg ctttgagggg gaaagggctg ggtggcctgg atggaagtcc 960
cttcccccag taaaagctac tgagagtttt tttgcccctg ctgcctgctg aggtacgggg 1020
acagtgggtc ctgccaggga cctccggact ctctcttcca cacaggtggc tcggggtgct 1080
cctcaagctt caggctagtg tgtgtgtgtg tgtgtgtgtg tgtgtgtgtg tgtgtggtga 1140
ggggcacagg ggaaggggtg atagtgataa tgggatgggg ggagggggag gcatttctgg 1200
ctcccagggc ctcctcggga gagtgaagga gactggagag accgacagga cggggcacgc 1260
ggccgaaaca ggggagcggg gtccgggtgg caccggcacg cactgcgcga gggccgggtg 1320
cgcagccatt cgctgcatgg ttagcggccg ctcccagggg cccggcggga cacacagtag 1380
gtggctcgga gaccactgcg tagccggcgg cggaagcggc tgggcccgga ggcggagacc 1440
cagacccgcc agactcgggc gctccaggcg gtcggggaag cccaggctcg gggtctcccg 1500
gcccgcagag tttgcagccc taggcgcccc catccccgcg cgtccgggac cggggagcgc 1560
gcgaggggac aggccgggga tagggggagg cgcccagacc ggccgccccc gcccctcccg 1620
cgggtcccca gacccctccc ctagcccaga ccctcaatcc cgaacccgtc ggccgcccct 1680
ccccggccgc cccgcccccg ccccgggctg catccaggcc ccagttccca gccgggggct 1740
gcgggctccc gccgcccgcc ccgcgctccc ccctcgcgcc cggtgcggcg ccccccgccc 1800
ccacccctcc ccgccctccc acctccccac ccaccccgcc agcccgtgac cctcccgccc 1860
gctccggctc ccgagcagcc agcggccacc ggctccggca gcggcgcaca gcgattcggt 1920
gcggcgcggc gagcacgacg ttccacggga cccgcggagc cgcgtcgtga tcgccgccgg 1980
cctcccgcac ccgcaccctc tccgctcgcg ccctgctcag cgcgtcctcc cgcggcggcc 2040
cgcgggacgg cgtgacccgc cgggctctcg gtgccccggg gccgcgcgcc atg ggc 2096
agc ccc cgc tcc gcg ctg agc tgc ct gtgagtaccg cgccgccctg 2142
ccccgccacc cgccccccgc gcccctcgcc tcacccgcgc ctctctctcc cgcccgcttt 2202
tgtctcccac ag g ctg ttg cac ttg ctg gtc ctc tgc ctc caa gcc cag 2251
gtgaggaggg gtgcgcggag gcgggggccg ggcgcgccgg tgtgagaccc gggtgggcag 2311
cgccggtcgg gggcaccggg actgactctg cggccggcgc gggagggctg agggcacctt 2371
agaaacccag ccccgagcca ccccgaggag ggagctgagg cacagagagg tagcacccct 2431
cctgaggtca cacagcgagt gagtggccag gatagaaacg aaggtctcgg agcccagcac 2491
tgtcccccat gcatcctcgc cggctggggg catagggaac acccagccgc cgaggaaggg 2551
ggcagccgcg gccaggggat ggatgttcga tgccagggga agccgggtga ctgcagcaga 2611
gaccctctca gcgcccctcg ggggaggctt gtggccgatt tggcccaatg atcgggtgcc 2671
caggttccct gcactctcaa catttgctcc gtaaatttgt ctttataaat gtcaggggtc 2731
ggggagggct gcctccctac ttaaaagcgc cctgctcctc tag gaa ggc ccg ggc 2786
agg ggc cct gcg ctg ggc agg gag ctc gct tcc ctg ttc cgg gct ggc 2834
cgg gag ccc cag ggt gtc tcc caa cag gtgggtccag cttctccctg 2881
gggctcgttc atcctgggct gggtctgccc gacttgcgtg ggtgggggat ggtggcctgg 2941
gctggcatgt tgggggaacc cccagcacct gctggcggct tggggcagtg agggggacgc 3001
agggtgatgg ggccactcgg gcccctgggc ggagtagcat tataatggtg tattgtgtat 3061
ttttcaattt cctaaag gta act gtt cag tcc tca cct aat ttt aca cag 3111
cat gtg agg gag cag agc ctg gtg acg gat cag ctc agc cgc cgc ctc 3159
atc cgg acc tac caa ctc tac agc cgc acc agc ggg aag cac gtg cag 3207
gtc ctg gcc aac aag cgc atc aac gcc atg gca gag gac ggc gac ccc 3255
ttc g gtaaggcgcg ctgaaggtag cttgtgggat gcgcggcgcc tggaccacca 3309
gtcccaactg cactggccgg gggcctgcag ggtccccagc tcagaaggga aggagttaag 3369
gcagaaaggt gtcttgaggg tcagctgggg aagagagggc tagggaaggg gcctcgggga 3429
gtacttggcc acaggtgccc ccttccctgc ccagcaaatg aatgaatcta ggaatctccc 3489
tttacacctc cccccttcat acactcctcc ccagctgcag gtggaggcag ggggttcagg 3549
gggagggggc ctaggtttaa ggcaagcgct tgaaagagga tatgtcgcca gggcccacaa 3609
aaggagagtg ggaaagagaa gggagagaaa tcccctcaac tctcccagtt ggaaaaggag 3669
gttgctggga acccttaaat actttaatcg gatgggtcaa tttacacgtg gaaactgggc 3729
ttggaagggg gctctccctg ctccattccc tggggaaccc aggcggctgg ggcagggctc 3789
ccggctccct gtggggtggc aagtgcggag agctactgcc ggcagagagg ctgaggactg 3849
attgatttct aaaacattca atattcctgc cttgaaacgg ggccaatttc cagagtcctg 3909
gatgtaccaa gagaaaagca agccctctgg gcggctggtg ttgtttttag agagcgatta 3969
ttgtgagttt aaaaaccttt aaataatgac cacgttaaac aagcatgaaa ggagagggga 4029
agactctatt aaaatgtaat aaaaaggtga ggcattttaa gagctaagaa gaaaaatgaa 4089
gaatgatata aaacagaaaa agaacgagct ttttttcaga tgtggagttt gaaaggagtt 4149
gcaagtctgt aaatgttaaa aaagaggttc aagattgttc tctctgaatc tctggagaga 4209
cccctgcgtc tggcagagcg attaaaactc ttcttacttt cacctttttt aacatttcct 4269
gaatactttc tcctcttctt gagccagttt gggggatcga gtatgtctcg ctgcattttt 4329
tctgcatcga cgtagctgac ttcccttcaa agctggagga gacataattt tcctgggata 4389
aatctgtcac tggggtggaa gaataggttt gaaaatgtta agctttccaa aagcccggtc 4449
gggagagctg ccgtaaactg cggccccagc tccccagatc ggagcagatt caaccgacat 4509
gggagagagc gggctttctg gggtgggggt gggggcggcc tgcctggtgg ccgagctccc 4569
ctactgggct ggcagagcag gcctaccatc cctttgtccc tccaaattag ctgcttgctg 4629
cccttctcag cttctaagga gaggagacga atgcctggct cttccagggt ttgctctgtc 4689
agagcaaatg gcccagcatc cttggatttc ggggagggaa gcagaagtca agctggtgtg 4749
ggggaattca tcttcagaca tcagaggaag cctgatgtcc acttctctcc gtggccatat 4809
aagaggtttc ccgccttccc tagtccctgc cccacagcag gataggagct ggaggcccta 4869
agaacagagg ctcagggtgt tcagccagga caggagtagt ccggggagaa tagcagagag 4929
gtctcaatgg cttctttacc ggccccagga agatgcagag gcacctactt catccctgcc 4989
tgccgcatct ggccagaatc cccagagtgg catcctctca ctcctccagt cagcatcctt 5049
cctggccctt aaaaatagct gcctcttgct gtggaaggca gagcgggaga gggatgagca 5109
gactggctag gtccaggagg aggtgggcag gcagggaggt gacagctgca tttccctgct 5169
gacttggggc caaaccgggg gcttggccac tggtcccagc actgagcctg tgtgaagcgg 5229
ggaacattac aagctgagca gtgatgactg acttgctgac ctcgatgccc caaacccctg 5289
ccatgggttt cagggacaga ggaggtgcca tatgtcaatg caggctaagt ccaagaactg 5349
ctttctcagg gaggggagag gctgggctct gcggcaggga cggcaggaga gccagcgcct 5409
tctgtccctt cctaaacagt gaagtcaccc tcactacttt cttgaggaca gcctataaga 5469
gatgattgca cagagaggtg aacaggacag gcttctgtgg caagttggct gtctaagggg 5529
aggggaatgg gaaaggaggc atagaattca gccctgcctc ctggcctcca gtctaagagg 5589
ccagcaggca ggattctccc tcctcctcac ccacagaagc tatttggtca ctgggctgag 5649
aagcccagct gtgatgaggc tgaaagagaa aagacaattg agttgttgat ttctagacac 5709
tgggctgggg accctggtga ctgggaagcc acagggaaga ggcaggatcc tcctggcagg 5769
tgtctgaggt tctgggaaag cagcctgctt tgggctgtgt gtttagggag agctgtcccc 5829
atccctagca ccttctcagc agagcagagc ctggtgatgt tgaggggctg ggggtggcca 5889
aagctgtgag ttagaaccca aagtggcaga aacaggagac aatgctttca tctttggcct 5949
tgggactgga ggtcacccct tcacccaagt gtttgtaatg acctgtgaga cacgcacaca 6009
taatatttgg agttggaagg atcctcggag atcttttgat tagctatgga actctgattc 6069
aattaaattt tacttagatc ctcaatctaa gagacatcac tgtctggttt aaaaagaggg 6129
tggaaggaat ggcacttcag ccttttcaat ttgatagtcc caagacctct cttgagaacc 6189
cagtttgaaa accataatcc cgtccactgc cctctgtaaa cccaggggtg gggcccgagg 6249
tccagaaaag ccctttctct gggttgcaca gcatgttagt agctgggctg gagctggagt 6309
ccatgttttg ggttcctggt ctttggagca gttgctgctg ggctgtgaca agtgtgtggg 6369
ccagctgggg tcagggatct gccaatagcc atcctgctac cttctctgac ag ca aag 6426
ctc atc gtg gag acg gac acc ttt gga agc aga gtt cga gtc cga gga 6474
gcc gag acg ggc ctc tac atc tgc atg aac aag aag ggg aag ctg atc 6522
gcc aag gtgaggcccc agcaggcagg tggggtccgc gggacctagg gagtggcttg 6578
ggctggctgg actcctccaa gacagtcctg ggctctgatc ccaacaaggt aggcagaagg 6638
ccacagggcc atcttggggt tacctcacga ttgggtgcag gagtgaggga gaaggaagct 6698
gtttgagaga tgaaaactat aaaaggcgaa ggaggaagaa tgaaggctag ggaaggggct 6758
ggggggtggt ggttggtaaa ttccatatat ctttttaaca aatcgccaaa ttgctttgct 6818
attatgtcca attacatggt accagcattt ggaatgttca gtaagccgca ggcccagccc 6878
ctcggccgag ctctgaaatg gctccattag tcacggctcc tctccagcct cgagctcccc 6938
acttcctggg cttctggggc tggggtctta gcatcttctc ccaggcctcc cctcccccat 6998
agggtggctg ccctggggcc agggaaccga agtcctgggg gggtgagagg ggcaggtggg 7058
gagacgggtg gccagactgg tgggcaggag gccagagcag gccaggctct gggcccctct 7118
ctctgtcttt ctgcgttggg gcccagcccc tccgtagaca accatgtgtc actgctgcct 7178
gggaggacag gaagttgccg ggtgggctgc gagttgtgag ggattagaga gcgggtgccc 7238
aggcaggggg tggggctgcg gctcctgccc acctcgccat ctgctggggt gcccacctgc 7298
tgtctggggc cgctcgccct ctgcctctgc tgggggggct ctgtaacgtg gtgtctggct 7358
cccctacctg cag agc aac ggc aaa ggc aag gac tgc gtc ttc acg gag 7407
att gtg ctg gag aac aac tac aca gcg ctg cag aat gcc aag tac gag 7455
ggc tgg tac atg gcc ttc acc cgc aag ggc cgg ccc cgc aag ggc tcc 7503
aag acg cgg cag cac cag cgt gag gtc cac ttc atg aag cgg ctg ccc 7551
cgg ggc cac cac acc acc gag cag agc ctg cgc ttc gag ttc ctc aac 7599
tac ccg ccc ttc acg cgc agc ctg cgc ggc agc cag agg act tgg gcc 7647
ccc gag ccc cga taggtgctgc ctggccctcc ccacaatgcc agaccgcaga 7699
gaggctcatc ctgtagggca cccaaaactc aagcaagatg agctgtgcgc tgctctgcag 7759
gctggggagg tgctggggga gccctgggtt ccggttgttg atattgtttg ctgttgggtt 7819
tttgctgttt tttttttttt tttttttttt aaaacaaaag agaggctcta tttttgtatt 7879
ccacttggct gtggtgtctg tcttcttaac tctcagaaag ctccattagt ggcctagact 7939
gggattccgg ctgggggttt gcgggggtgg ggggctttct ctagcctgtg ctgctgaggc 7999
cccagtacct ccagggccag ttggctgggc agccagggac tccactgcac ccccaggtgg 8059
ggcagggagg aaaggactgt gacatagggc agtcctctta gaagtgggta tcagactggt 8119
ggctattaaa tgattgaaat atttatttaa cttgcatatt aaaaatgtgt gctggagagt 8179
gagtcctgcc ggggtcagcc cctccctcca accttgcccc agctggtggg cggctgggag 8239
acgcagatga ccaggtgcca gctctgacca cagcctccct ccagcctaaa gacacctgcc 8299
tgtcaaccat ccccatcact gtcacttgag gggttttcct gcaaggacag aagcagggaa 8359
aggggcaaga agaggctctt agctagtcct tggagctctc agatgtgtac ctcctagcac 8419
tttacagagg tcattgctaa cacttcccca ggccacctca gggccagaaa taatggatgt 8479
gctagggcta gagctgtaat catggattta atcctcttaa aaagtgcttc tctgagtgcc 8539
taggtccatg tgggagacag gttggagatt ccagaacttg ctctttctga gactcaggct 8599
ccagaaaatg aaagaaaaga gcagctgcca gggtccaagg tgggggcata ttggaggggg 8659
accaccaaga ctggtgttga caatggtgat gtgggacaag tgttaacctt gggtgatatg 8719
gtgagatagc tgtgggcaga aagcactgag ctgaggtgcg gtgaggagcc tggggaactg 8779
tcttccagga agaggctgcc cacctcggag gatgggcctg gcgggagagg agctgggcac 8839
cggatggcac cagaagggaa gctcataggc ctagcgcaga actaaaggca gtcatagcct 8899
tggggagaag caggaggccg tatgtggagg gagggagggc tgctgtggga gtggtggagc 8959
aggtcatggt gtgggcagag aagggaatgg gcaagggtgc aggtgtgtgt ttgcgtgtgg 9019
actggtgaga ctggtgtcct gccacaccga gggagagccc aggccccacg gcagtttcct 9079
gagtgcagag ctggcccagg cttcatcgct gaggcctccc attagggctg ctcctgcttc 9139
cttccttgtg gatgccctgg gctggtccca cagcccagct actgagccag tctagaaacc 9199
tcgctcctct aggactctgt ccattggcat ctcctggggt agggagcaac ccaaggcttc 9259
ctcccacagt ttcaggccca agtcccaggt ttagcccacc ccggcagatc aggaatttcg 9319
aggcttagtg ggaagcccgg cagtaaaggc tggcctgtcc agccacctct cgtggcctgc 9379
cacaggcggt gaggggctct ggagagagtg ggtttctgct ggtgcgtaga cactggatgt 9439
agcgaagggg ccctagatgt ctctcttcca gttcccccag ctcttagcgc ctcctcctct 9499
gctcccttcc ccctttaatt aattctgggt aagcagcctg gctttattgt gcaaggagct 9559
ggggtctcac tgtgggaaaa gtcacacctt ctgagcagct tctgatgcca tgcaaatgaa 9619
gggaccgtcc aggaaacgct ttcatctgca cggtctcctg tccagcccct agcccagtaa 9679
ttacccacat ttcatttgtt tgagagcaat tcctg 9714

<210> 4

<211> 244

<212> PRT

<213> human

<400> 4

<210> 5
<211> 1238
<212> DNA
<213> human

<220>
<221> CDS
<222> (13)..(663)
<223>

<300>

<308>   NM003867

<309>   2007-01-21

<400>   5

acctctccag cg atg gga gcc gcc cgc ctg ctg ccc aac ctc act ctg tgc   51

Met Gly Ala Ala Arg Leu Leu Pro Asn Leu Thr Leu Cys

1               5                   10

tta cag ctg ctg att ctc tgc tgt caa act cag ggg gag aat cac ccg   99

Leu Gln Leu Leu Ile Leu Cys Cys Gln Thr Gln Gly Glu Asn His Pro

15                  20                  25

tct cct aat ttt aac cag tac gtg agg gac cag ggc gcc atg acc gac   147

Ser Pro Asn Phe Asn Gln Tyr Val Arg Asp Gln Gly Ala Met Thr Asp

30                  35                  40                  45

cag ctg agc agg cgg cag atc cgc gag tac caa ctc tac agc agg acc   195

Gln Leu Ser Arg Arg Gln Ile Arg Glu Tyr Gln Leu Tyr Ser Arg Thr

                50                  55                  60

agt ggc aag cac gtg cag gtc acc ggg cgt cgc atc tcc gcc acc gcc   243

Ser Gly Lys His Val Gln Val Thr Gly Arg Arg Ile Ser Ala Thr Ala

            65                  70                  75

gag gac ggc aac aag ttt gcc aag ctc ata gtg gag acg gac acg ttt   291

Glu Asp Gly Asn Lys Phe Ala Lys Leu Ile Val Glu Thr Asp Thr Phe

        80                  85                  90

ggc agc cgg gtt cgc atc aaa ggg gct gag agt gag aag tac atc tgt 339
Gly Ser Arg Val Arg Ile Lys Gly Ala Glu Ser Glu Lys Tyr Ile Cys
    95                  100                 105

atg aac aag agg ggc aag ctc atc ggg aag ccc agc ggg aag agc aaa 387
Met Asn Lys Arg Gly Lys Leu Ile Gly Lys Pro Ser Gly Lys Ser Lys
110                 115                 120                 125

gac tgc gtg ttc acg gag atc gtg ctg gag aac aac tat acg gcc ttc 435
Asp Cys Val Phe Thr Glu Ile Val Leu Glu Asn Asn Tyr Thr Ala Phe
                130                 135                 140

cag aac gcc cgg cac gag ggc tgg ttc atg gcc ttc acg cgg cag ggg 483
Gln Asn Ala Arg His Glu Gly Trp Phe Met Ala Phe Thr Arg Gln Gly
            145                 150                 155

cgg ccc cgc cag gct tcc cgc agc cgc cag aac cag cgc gag gcc cac 531
Arg Pro Arg Gln Ala Ser Arg Ser Arg Gln Asn Gln Arg Glu Ala His
        160                 165                 170

ttc atc aag cgc ctc tac caa ggc cag ctg ccc ttc ccc aac cac gcc 579
Phe Ile Lys Arg Leu Tyr Gln Gly Gln Leu Pro Phe Pro Asn His Ala
    175                 180                 185

gag aag cag aag cag ttc gag ttt gtg ggc tcc gcc ccc acc cgc cgg 627
Glu Lys Gln Lys Gln Phe Glu Phe Val Gly Ser Ala Pro Thr Arg Arg

190                 195                 200                 205

acc aag cgc aca cgg cgg ccc cag ccc ctc acg tag tctgggaggc 673
Thr Lys Arg Thr Arg Arg Pro Gln Pro Leu Thr
                210                 215

agggggcagc agcccctggg ccgcctcccc acccctttcc cttcttaatc caaggactgg 733

gctggggtgg cgggagggga gccagatccc cgagggagga ccctgagggc cgcgaagcat 793

ccgagccccc agctgggaag gggcaggccg gtgccccagg ggcggctggc acagtgcccc 853

cttcccggac gggtggcagg ccctggagag gaactgagtg tcaccctgat ctcaggccac 913

cagcctctgc cggcctccca gccgggctcc tgaagcccgc tgaaaggtca gcgactgaag 973

gccttgcaga caaccgtctg gaggtggctg tcctcaaaat ctgcttctcg gatctccctc 1033

agtctgcccc cagcccccaa actcctcctg gctagactgt aggaagggac ttttgtttgt 1093

ttgtttgttt caggaaaaaa gaaagggaga gagaggaaaa tagagggttg tccactcctc 1153

acattccacg acccaggcct gcaccccacc cccaactccc agccccggaa taaaaccatt 1213

ttcctgcaaa aaaaaaaaaa aaaaa 1238

<210> 6

<211> 216

<212> PRT

<213> human

<400> 6

Met Gly Ala Ala Arg Leu Leu Pro Asn Leu Thr Leu Cys Leu Gln Leu
1               5                   10                  15

Leu Ile Leu Cys Cys Gln Thr Gln Gly Glu Asn His Pro Ser Pro Asn
            20                  25                  30

Phe Asn Gln Tyr Val Arg Asp Gln Gly Ala Met Thr Asp Gln Leu Ser
        35                  40                  45

Arg Arg Gln Ile Arg Glu Tyr Gln Leu Tyr Ser Arg Thr Ser Gly Lys
    50                  55                  60

His Val Gln Val Thr Gly Arg Arg Ile Ser Ala Thr Ala Glu Asp Gly
65                  70                  75                  80

Asn Lys Phe Ala Lys Leu Ile Val Glu Thr Asp Thr Phe Gly Ser Arg
                85                  90                  95

Val Arg Ile Lys Gly Ala Glu Ser Glu Lys Tyr Ile Cys Met Asn Lys
            100                 105                 110
Arg Gly Lys Leu Ile Gly Lys Pro Ser Gly Lys Ser Lys Asp Cys Val
        115                 120                 125
Phe Thr Glu Ile Val Leu Glu Asn Asn Tyr Thr Ala Phe Gln Asn Ala
    130                 135                 140
Arg His Glu Gly Trp Phe Met Ala Phe Thr Arg Gln Gly Arg Pro Arg
145                 150                 155                 160
Gln Ala Ser Arg Ser Arg Gln Asn Gln Arg Glu Ala His Phe Ile Lys
                165                 170                 175
Arg Leu Tyr Gln Gly Gln Leu Pro Phe Pro Asn His Ala Glu Lys Gln
            180                 185                 190
Lys Gln Phe Glu Phe Val Gly Ser Ala Pro Thr Arg Arg Thr Lys Arg
        195                 200                 205

Thr Arg Arg Pro Gln Pro Leu Thr
    210                 215

## Description

### Technical Field

The present invention relates to a tooth root formation promoting factor and a method for promotion of tooth root formation, which are useful in various aspects of dental therapy where formation (elongation) of tooth roots is desired.

### Background Art

Children, among others, often experience tooth avulsion due to, for example, accidental injuries. Avulsed teeth are recommended to be reduced and fixed as soon as possible. Teeth have thereinside dental pulp tissue rich in nerves and blood vessels and avulsed teeth, in most cases, involve degeneration and devitalization of the dental pulp tissue. In this case, according to a conventional treatment method, the devitalized necrotic tissue is removed and the prepared root canal is filled with calcium hydroxide (see, for example, Non-Patent Literature 1).

Conventionally in dentistry, apexogenesis refers to a treatment which intends to elongate tooth root and apical closure, whereas apexification refers to a treatment which intends only to close tooth root end using hard tissue. In other words, a treatment which has attained tooth root elongation is called apexogenesis, whereas a treatment which has not attained tooth root elongation is called apexification. The larger the area of a surface where tooth roots face alveolar bone supporting them, the longer and more stable teeth reside in the mouth. Thus, the avulsed teeth, in particular the avulsed root-forming teeth of children, are treated in an attempt to attain apexogenesis; i.e., tooth root elongation.

However, according to the above conventional treatment method, such cases are often encountered that even apexification cannot be attained (needless to say, apexogenesis cannot be attained).

In recent years, there has been positively performed tooth autotransplantation in which an autogenous tooth is transplanted into a portion missing from a tooth. In the tooth autotransplantation, a third molar (a wisdom tooth) or a root-forming third molar is often used. In use of the root-forming third molar, whether or not the transplanted tooth has the same functions as an intrinsic tooth depends on how long the tooth root thereof elongates or how firmly the elongated tooth root is supported by periodontal alveolar bone and periodontal ligament. In view of this, it is required to find a new approach for elongating the root of the transplanted tooth or for positively inducing the periodontal tissue to support the tooth root.

One developed technique includes applying FGF2 onto, for example, a tooth root surface for promoting tooth root formation (elongation) (Patent Literature 1 and 2). Meanwhile, various factors are thought to be involved in tooth root formation *in vivo.* Thus, demand has arisen for identification of more useful factors in various aspects of dental therapy; e.g., tooth autotransplantation, and treatments for avulsed teeth and periodontal disease.
Non-Patent Literature 1: Int. Dent. J. 2005, Oct.; 55(5): 293-301
Patent Literature 1: Japanese Patent Application Laid-Open No. 07-17876
Patent Literature 2: International Publication No. WO2003/082321

### Disclosure of Invention

An object of the present invention is to solve the problems pertinent in the art and to achieve the following objects. Specifically, an object of the present invention is to provide a tooth root formation promoting factor and a method for promotion of tooth root formation, which are useful in various aspects of dental therapy.

The present inventors carried out extensive research and, as a result, obtained the following findings. Specifically, the present inventors studied on BMP2, FGF2 and FGF18 (known physiologically active substances having bone induction activity) for their tooth root formation promoting activities (tooth root elongation inducing activities) to a root-forming tooth of a mammalian, and have found that FGF18, among others, has remarkably excellent tooth root formation promoting activity. Also, the present inventors, through experiments on functional replacements using an FGF18 knockout mouse, have found that FGF8 belonging to the same subfamily as FGF18 (i.e., the FGF8 subfamily) replaces the function of FGF18. From the above findings, the present inventors have conceived that proteins belonging to the FGF8 subfamily can be effectively used for tooth root elongation, and the present invention has been accomplished.

No reports have been presented on the fact that the proteins belonging to the FGF8 subfamily positively exhibit tooth root formation promoting activity, and this is a quite surprising fact. These proteins can provide a promising approach to dental disease, to which no existing treatment methods are effective and which are expected to attain apexogenesis (a treatment which intends to elongate tooth root and apical closure).

As used herein, "formation" of tooth roots refers not only to formation of new tooth roots but also to "elongation" of existing tooth roots, and "elongation" of tooth roots refers not only to elongation of existing tooth roots but also to "formation" of new tooth roots. Thus, "formation" and "elongation" are herein equivalent to each other, and this shall apply to expressions equivalent to any of these.

The present invention is based on the above findings obtained by the present inventors, and provides the followings.
<1> A tooth root formation promoting factor containing, a protein belonging to the FGF8 subfamily as an active ingredient.
<2> A method for promotion of tooth root formation including, administering a protein belonging to the FGF8 subfamily to a mammalian.

The present invention can solve the aforementioned problems, and provides a tooth root formation promoting factor and a method for promotion of tooth root formation, which are useful in various aspects of dental therapy.

### Brief Description of Drawings

FIG.1 shows photographs in relation to transplant culturing which includes subrenally transplanting extirpated teeth having embedded FGF18-impregnated beads.
FIG. 2 is a graph showing the length of the formed (elongated) tooth root, after transplant culturing, of each extirpated tooth with a physiologically active substance (BMP2, FGF2 or FGF18)-impregnated bead being embedded.
FIG. 3 is a photograph of an extirpated tooth having an embedded FGF18-impregnated bead, wherein the photograph is obtained using soft X-ray photography after transplant culturing.
FIG. 4 shows images of an extirpated tooth having an embedded FGF18-impregnated bead after transplant culturing, wherein the images are obtained after histological staining and the characters B, C, D, PL, CB and OB denote bone, cementum, dentine, periodontal ligament, cementoblasts and osteoblasts, respectively.
FIG. 5 is photographs showing GFP-positive bone formation in a GFP-mouse-derived tooth having an embedded FGF18-impregnated bead after transplant culturing.
FIG. 6 is photographs showing, after transplant culturing, extirpated teeth with physiologically active substance (FGF2 or FGF18)-impregnated beads being embedded, periodontal bone and neovascularization.
FIG. 7 is an electrophoretic profile in relation to endogenous FGF18 expression in dental pulps (including tooth roots) of incisors of mice and rats which continue to form tooth roots.
FIG. 8 is photographs showing, after transplant culturing, extirpated teeth having embedded physiologically active substance (FGF8)-impregnated beads, periodontal bone and neovascularization, wherein white lines indicate profiles of elongated tooth roots.

### Best Mode for Carrying Out the Invention

### (Tooth root formation promoting factors)

A tooth root formation promoting factor of the present invention contains proteins belonging to the FGF8 subfamily (FGF8 subfamily proteins) as an active ingredient and, if necessary, appropriately contains other ingredients.

### <Proteins belonging to the FGF8 subfamily>

Fibroblast growth factors (FGFs) are classified into seven subfamilies according to structural similarity. FGF18, which belongs to the FGF8 subfamily including FGF8 and FGF17, is known as a physiologically active substance having bone formation stimulating activity. However, no reports have been presented concerning its effects on tooth roots.

The present inventors have first found that FGF18 has excellent tooth root formation promoting activity. Also, through experiments on functional replacements using FGF18 knockout mice, it has been found that FGF8 belonging to the same subfamily as FGF18 (i.e., the FGF8 subfamily) replaces the functions of FGF18. Thus, the FGF8 subfamily proteins may be used as an active ingredient in the present tooth root formation promoting factors.

The FGF8 subfamily proteins are not particularly limited and appropriately selected depending on the purpose. Examples include recombinant human FGF8 subfamily proteins, recombinant mouse FGF8 subfamily proteins, and those derived from other mammalians. When used in a human for treatment, the FGF8 subfamily proteins are particularly preferably derived from a human. Notably, in the sequence listing given below, SEQ ID Nos:1 and 2 correspond to human FGF18; SEQ ID Nos: 3 and 4 correspond to human FGF8; and SEQ ID Nos: 5 and 6 correspond to human FGF17.

The FGF8 subfamily proteins may be a purified extract derived from, for example, living organisms; or may be produced from microorganisms or culture cells with a known recombinant protein production technique. Also, in addition to the purified extract, an unpurified cell extract, etc. may be used as the FGF8 subfamily proteins.

Furthermore, the FGF8 subfamily proteins may be a commercially available one, and examples include those of, for example, PeproTech Co. and Sigma Co.

The FGF8 subfamily proteins are not particularly limited, so long as they have the intrinsic physiological activities thereof and have tooth root formation promoting activity. For example, there may be used polypeptides having an amino acid sequence substantially identical to those of naturally occurring FGF8 subfamily proteins. Notably, the polypeptides are those having the physiological activities of the naturally occurring FGF8 subfamily proteins and being identical thereto in amino acid sequence, except that several amino acids are substituted. Also, fragments of the polypeptides may be used as the FGF8 subfamily proteins, so long as they have the physiological activities of the naturally occurring FGF8 subfamily proteins and have tooth root formation promoting activity. Furthermore, the polypeptides or fragments may be modified at their N- and/ or C-terminuses, so long as they have the physiological activities of the naturally occurring FGF8 subfamily proteins and have tooth root formation promoting activity.

The FGF8 subfamily proteins may be used alone or in combination.

The FGF8 subfamily protein content of the tooth root formation promoting factors is not particularly limited, and can be appropriately determined in consideration of the selected dosage form of the tooth root formation promoting factors. The content is preferably 0.025% by mass to 3.0% by mass, more preferably 0.1% by mass to 1.0% by mass, particularly preferably 0.25% by mass to 0.5% by mass.

### <Other ingredients>

The tooth root formation promoting factors may be the FGF8 subfamily protein itself, or a product containing the FGF8 subfamily protein and other ingredients.

The other ingredients are not particularly limited and can be appropriately selected depending on the purpose so as not to impede the effects of the present invention. Examples thereof include pharmaceutically acceptable carriers. The pharmaceutically acceptable carriers are not particularly limited and can be appropriately selected in consideration of, for example, the selected dosage form of the tooth root formation promoting factors. Examples thereof include solvents, isotonicity agents, emulsifiers, suspending agents, stabilizers and fillers, which are generally used in drugs for use in dental therapies.

In particular, heparin-containing absorbable beads or gel is preferred as the pharmaceutically acceptable carrier, since the FGF8 subfamily protein has an affinity to heparin. The heparin-containing absorbable beads or gel may be commercially available from, for example, Sigma Co.

The other ingredients content is not particularly limited and can be appropriately determined depending on the purpose so as not to impede the effects of the present invention. For example, the content may be appropriately determined so as to attain the desired FGF8 subfamily protein (active ingredient) content of the tooth root formation promoting factors.

### <Dosage form>

The dosage form is not particularly limited and can be appropriately selected depending on the purpose from known dosage forms. For example, the FGF8 subfamily protein and the carrier appropriately selected from the above carriers are formed into an external preparation (e.g., liquid, emulsion, or a gelling agent), an impregnating agent, an adhesive preparation, injection or powder. The method for forming the protein and carrier into any of the above dosage forms is not particularly limited and can be appropriately selected depending on the purpose from known forming methods.

In particular, preferred are heparin-containing absorbable beads or gel which is impregnated with the FGF8 subfamily protein. The FGF8 subfamily protein-impregnated heparin-containing absorbable beads or gel can provide tooth root formation promoting factors having controlled-release properties. This is advantageous in that the tooth root formation promoting factor is not lost from an application site in a short time due to outflow, etc., and that the FGF8 subfamily protein serving as an active ingredient can continue to act on the application site for a relatively long period of time (preferably for two to three days).

The method for forming the FGF8 subfamily protein-impregnated heparin-containing absorbable beads is not particularly limited. For example, they can be formed as follows: the FGF8 subfamily protein is diluted with phosphate buffered saline (PBS); and the resultant solution was added to commercially available heparin-containing absorbable beads; and the resultant beads are incubated (shown in Examples given below).

The method for forming the FGF8 subfamily protein-impregnated heparin-containing absorbable gel is not particularly limited. For example, it can be formed as follows: a heparin solution is added to collagen gel formed predominantly of type I collagen; and heparin is electrostatically bonded to the type I collagen in PBS, followed by incubation (see, for example, Biochim. Biophys. Acta. (1986) 882:1-5).

The tooth root formation promoting factor can be provided with desired controlled-release properties by appropriately determining the amount of a solution of the FGF8 subfamily protein and the employed carrier. The controlled-release tooth root formation promoting factor enables the FGF8 subfamily protein, serving as an active ingredient, to act on an application site for a period of time that is determined depending on the symptoms, etc. of a patient in need thereof.

### (Method for promotion of tooth root formation)

A method for promotion of tooth root formation of the present invention includes administering the FGF8 subfamily proteins to a mammalian and, if necessary, includes other steps.

In the method for promotion of tooth root formation, the form of the FGF8 subfamily protein administered to a mammalian is not particularly limited. The FGF8 subfamily protein may be administered as is, or may be administered in the form of FGF8 subfamily protein-impregnated heparin-containing absorbable beads or gel (i.e., a preferred dosage form of the tooth root formation promoting factors). Use of the FGF8 subfamily protein-impregnated heparin-containing absorbable beads or gel is particularly preferred, since the FGF8 subfamily protein can act on an administration site thereof in a mammalian for a relatively long period of time.

### <Mammalian>

The mammalian to which the tooth root formation promoting factor (FGF8 subfamily protein) is administered is not particularly limited and can be appropriately selected depending on the purpose. Examples thereof include humans, pet animals, livestock animals and experimental animals (e.g., monkeys, bovines, pigs, mice, rats, dogs and cats).

### <Administration>

The site to which the tooth root formation promoting factor (FGF8 subfamily protein) is administered is not particularly limited, so long as formation of a tooth root of interest can be promoted. For example, the promoting factor (protein) can be applied to a dental pulp, a tooth root surface, an alveolar bone surface, or sites in the vicinity thereof. The administration method of the tooth root formation promoting factor (FGF8 subfamily protein) is not particularly limited and can be appropriately selected from known administration methods in consideration of, for example, the dosage form of the tooth root formation promoting factor. For example, the promoting factor (protein) may be embedded in or injected to a dental pulp; or may be coated on a tooth root surface or an alveolar bone surface.

The administration dose of the tooth root formation promoting factor (FGF8 subfamily protein) is not particularly limited and can be appropriately determined in consideration of various factors such as the symptoms, constitution, age and body weight of a target subject, and his/her concomitant medication. For example, the promoting factor (protein) is preferably administered to a human adult at a dose of 0.01 mg to 1.0 mg, more preferably 0.05 mg to 0.5 mg, particularly preferably 0.1 mg to 0.3 mg, wherein each administration dose is a dose at which the promoting factor (protein) is singly administered to one site, and is reduced to the amount of the FGF8 subfamily protein (active ingredient). Also, the dose at which the promoting factor (protein) is administered to the other mammalians is not particularly limited and can be appropriately determined in consideration of the above various factors. In general, the administration dose for the other mammalians may be determined by reducing the administration dose for the human adult on the basis of the body weight. Particularly preferably, the promoting factor (protein) is administered to a target subject at a dose of 2.5 µg/kg. Furthermore, the number at which the promoting factor (protein) is administered per day is not particularly limited and can be appropriately determined in consideration of the above various factors.

### [Effects]

The present tooth root formation promoting factor containing, as an active ingredient, the FGF8 subfamily proteins have excellent tooth root formation promoting activity. Thus, when administered to a mammalian, the present promoting factor can promote formation of tooth roots thereof. Also, the present tooth root formation promoting factor can be provided with desired controlled-release properties by appropriately determining the employed carrier and the amount of the FGF8 subfamily proteins serving as an active ingredient, and the controlled-release tooth root formation promoting factor can act on an application site for a desired period of time. Furthermore, the present tooth root formation promoting factor has excellent safety, since it employs safe FGF8 subfamily proteins as an active ingredient.

Thus, the present tooth root formation promoting factor and the present method for promotion of tooth root formation, which employ the FGF8 subfamily protein, can be expected to be clinically applied to various aspects of dental therapy where promotion of tooth root formation is desired. Conceivably, the present promoting factor and method are particularly advantageously used as the followings (1) to (7):
(1) use thereof in pediatric dentistry can induce tooth root elongation in root-forming teeth, which have suffered from disorders caused by trauma or infection in the process of tooth root formation and have been prevented from elongating;
(2) use thereof in orthodontic dentistry can induce regeneration of tooth roots resorbed due to orthodontic force accompanying orthodontic therapy;
(3) use thereof in general dentistry can induce regeneration of tooth roots resorbed caused by lesions in root apices (e.g., radicular cyst);
(4) use thereof in general dentistry can induce regeneration of tooth root-periodontal tissue after intentional reimplantation for the treatment of, for example, root fracture;
(5) use thereof in oral surgery can promote formation of tooth root-periodontal tissue of autotransplanted supernumerary teeth (third molars (i.e., wisdom teeth));
(6) use thereof in regenerative medicine can be expected to induce regeneration of tooth root-periodontal tissue after autotransplantation of juvenile teeth which have been stored in a frozen state; and
(7) use thereof in general dentistry can be expected to induce regeneration of cementum, periodontal ligament and alveolar bone resorbed due to, for example, periodontal disease.

As shown in Examples given below, the present tooth root formation promoting factors and the present method for promotion of tooth root formation exhibit not only tooth root formation promoting activity but also an activity of promoting formation of periodontal tissues supporting tooth roots; e.g., periodontal ligament and alveolar bone. Thus, the present promoting factors and method are expected to be clinically applied to a wider variety of aspects of dental therapy.

Also, the present tooth root formation promoting factors and the present method for promotion of tooth root formation can be applied not only to apexogenesis therapy intending to elongate tooth root and closure root apex but also to apexification therapy intending only to close tooth root apices using hard tissue. Thus, the present promoting factors and method are expected to be used in a wider variety of clinical applications.

### Examples

The present invention will next be described by way of examples, which should not be construed as limiting the present invention thereto.

### (Example 1: FGF18)

### <Preparation of FGF18-impregnated heparin-containing absorbable beads>

FGF18-impregnated heparin-containing absorbable beads (a preferred embodiment of a tooth root formation promoting factor of the present invention) were prepared as follows.

FGF18 (product of PeproTech Co.) was diluted with Dulbecco's modified phosphate buffered saline (D-PBS) to a concentration of 250 µg/mL. Heparin acrylic beads (product of Sigma Co.), which had been provided as heparin-containing absorbable beads, were washed with phosphate buffered saline (PBS). The obtained FGF18-diluted solution was added to the thus-washed heparin acrylic beads, followed by incubation at 37°C for 45 minutes, whereby the heparin acrylic beads were impregnated with FGF18. The obtained FGF18-impregnated heparin acrylic beads were stored at 4°C before use. In use, after washed with D-PBS so as to remove excessive FGF18, the beads were set in dental pulps as described below.

### <Embedment in dental pulp>

Five-day-old C57BL/6 mice, at a stage when their tooth roots were about to be formed, were etherized, followed by decapitation. After removal of the mandibles, the mandibular first molars were extirpated therefrom together with the dental follicles. Thereafter, the FGF18-impregnated heparin acrylic beads were set in the dental pulps of some extirpated teeth (mandibular first molars) so that one bead was set in the dental pulp of one extirpated tooth.

Meanwhile, D-PBS alone was embedded in the dental pulps of other extirpated teeth (mandibular first molars), which were used as a negative control.

### <Transplant culturing>

The FGF18-impregnated heparin acrylic bead-embedded teeth and the D-PBS-embedded teeth (serving as a negative control) were subrenally transplanted into host mice, followed by culturing. Three weeks after, each of the host mice was killed with cervical dislocation, and the kidney was removed to recover the cultured teeth (transplanted teeth). Notably, the host mice were 10- to 15-week-old C57BL/6 male mice, since nude mice were not particularly required in this transplant culturing. This is because, among organs of an adult mouse, the kidney and the camera anterior bulbi are known to exhibit considerably lower immune response.

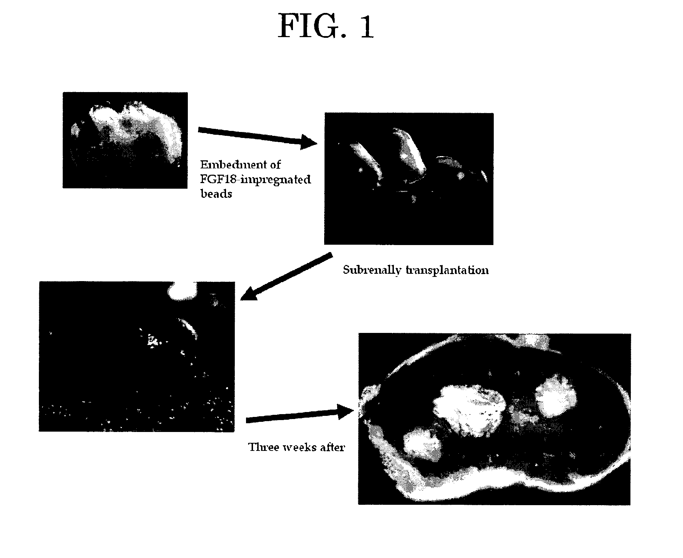

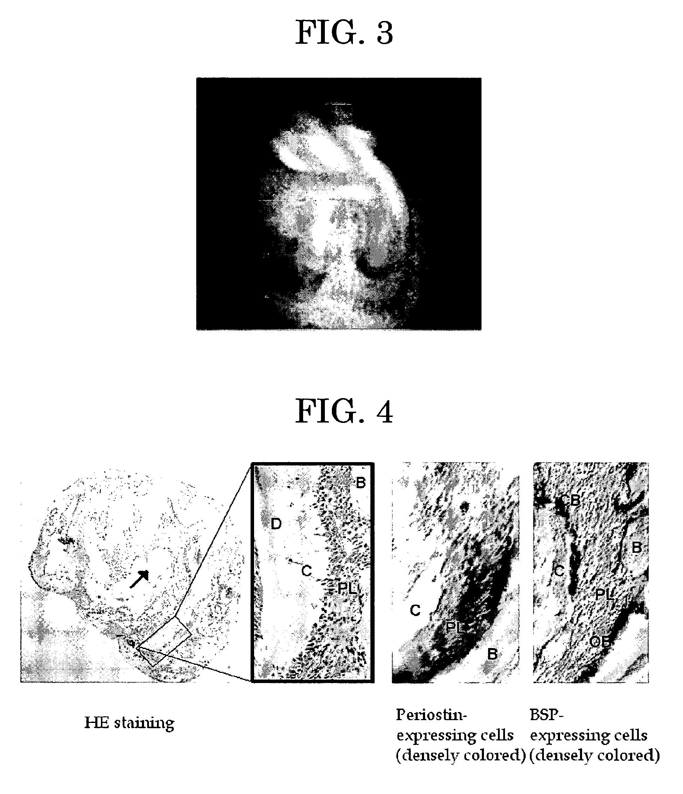

The recovered transplanted teeth were observed under a stereomicroscope for their tooth root formations (elongations). FIG.1 shows photographs in relation to the transplant culturing. FIGs. 3 to 6 show photographs of the transplanted teeth, periodontal bone and neovascularization.

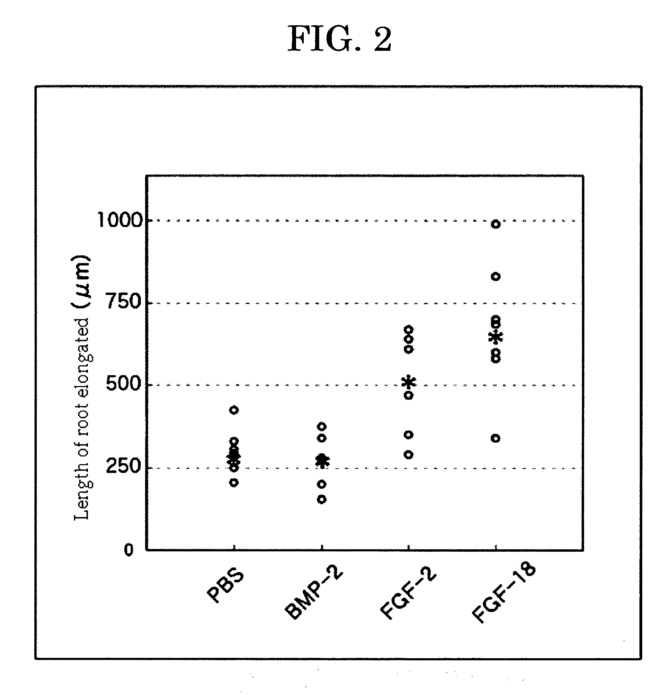

The formed (elongated) tooth roots of the recovered transplanted teeth were photographed with a stereofluorescence microscope system VBG-25 (product of KEYENCE CORPORATION) to obtain digital images thereof. The length (µm) of the formed (elongated) tooth roots was measured, while observing the obtained digital images, using an automatic measuring software attached to the system. The results are shown in FIG. 2.

### (Comparative Example 1: BMP2)

The procedure of Example 1 was repeated, except that BMP2 (product of R&D Co.) was used instead of FGF18, the concentration of the diluted solution was changed to 500 ng/mL, and Affi-Gel agarose beads (product of Bio-Rad Laboratories, Inc.) were used instead of the heparin acrylic beads, to thereby prepare BMP2-impregnated agarose beads of Comparative Example 1.

Subsequently, similar to Example 1, the BMP2-impregnated agarose beads of Comparative Example 1 were embedded in the dental pulps, followed by transplant culturing. Also, the teeth obtained after transplant culturing were measured for the length of the formed (elongated) tooth roots. The results are shown in FIG. 2.

### (Comparative Example 2: FGF2)

The procedure of Example 1 was repeated, except that FGF2 (product of PeproTech Co.) was used instead of FGF18, to thereby prepare FGF2-impregnated heparin acrylic beads of Comparative Example 2.

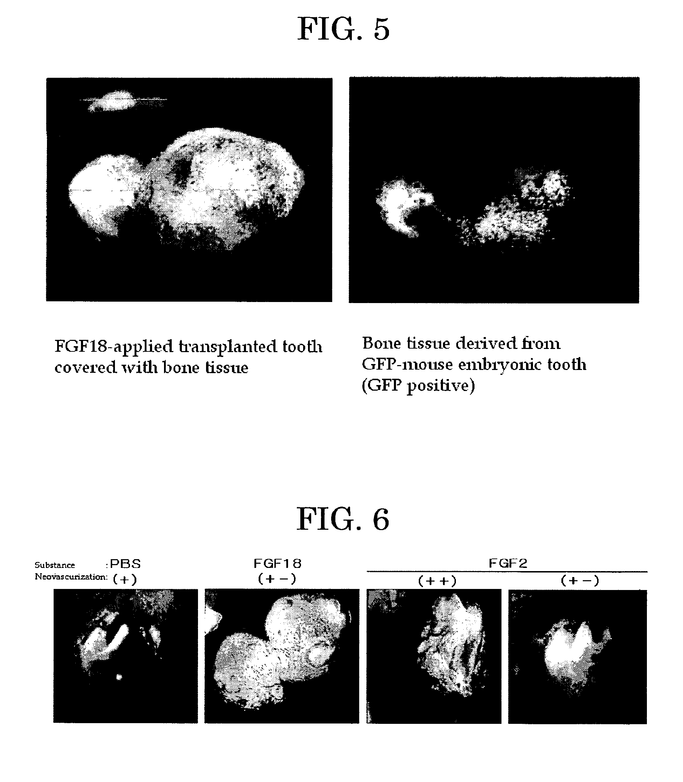

Subsequently, similar to Example 1, the FGF2-impregnated heparin acrylic beads of Comparative Example 2 were embedded in the dental pulps, followed by transplant culturing. Also, the teeth obtained after transplant culturing were measured for the length of the formed (elongated) tooth roots. The results are shown in FIG. 2. In addition, the teeth, periodontal bone and neovascularization were observed. The results are shown in FIG. 6.

### [Results (Example 1 and Comparative Examples 1 and 2)]

From the data shown in FIG. 2, the teeth of the negative control group using D-PBS alone (PBS in FIG. 2) and the experimental group using BMP2 (Comparative Example 1; BMP-2 in FIG. 2) were found to somewhat exhibit tooth root elongation. The teeth of the experimental group using FGF2 (Comparative Example 2; FGF-2 in FIG. 2) were found to exhibit tooth root elongation somewhat significantly greater than those of the negative control group and the BMP2-experimental group, but the length of the elongated tooth roots was found to be less than that of the experimental group using FGF18 (Example 1; FGF-18 in FIG. 2). Thus, the teeth of the FGF18-experimental group were found to exhibit tooth root elongation remarkably greater than those of any other experimental groups.

Notably, in FIG. 2, the symbol "o" corresponds to measurements for transplanted tooth samples of each group in this experiment, and the symbol "*" corresponds to mean values of the measurements.

The transplanted tooth samples of Example 1, which have been embedded with FGF18-impregnated heparin acrylic beads and exhibited promoted tooth root elongation, were found to exhibit bone formation around the formed tooth roots. The formed bone was subjected to soft X-ray photography to determine whether or not the tooth roots of the transplanted teeth elongated thereinside. From the obtained photographs, the tooth roots were found to elongate inside the formed bone (FIG. 3). In addition, through histological observation with HE staining, periodontal ligament-like fibroblasts were found between the formed tooth roots and the periodontal bone (a left-hand photograph of FIG. 4). In the left-hand photograph of FIG. 4, the embedded FGF18-impregnated heparin acrylic bead is indicated by an arrow. Furthermore, through *in situ* hybridization, these periodontal ligament-like fibroblasts were found to express the periostin gene, which is a marker gene of the periodontal ligament (a middle photograph of FIG. 4), and also, bone siaroprotein (BSP)-positive cells were found to be present on the surface of the bone around the tooth roots (a right-hand photograph of FIG. 4). In the middle and right-hand photographs of FIG. 4, densely colored are areas where cells expressing the periostin gene and producing the bone siaroprotein are present.

Separately, when FGF18 was applied together with an embryonic tooth of a green fluorescent protein (GFP)-mouse serving as a donor tooth, the bone formed around a transplanted tooth (a left-hand photograph of FIG. 5) was found to be derived from tissue thereof with a stereofluorescence microscope MZFLIII (product of Leica MICROSYSTEMS) (a right-hand photograph of FIG. 5).

From photographs shown in FIG. 6, the teeth of the negative control group using D-PBS alone (in FIG. 6, a photograph specified by PBS and neovascularization (+ -)) and the FGF2-experimental group that did not considerably exhibit neovascularization (Comparative Example 2; in FIG. 6, a photograph specified by FGF2 and neovascularization (+ -)) were found to somewhat exhibit tooth root elongation. The teeth of the FGF2-experimental group that considerably exhibited neovascularization (Comparative Example 2; in FIG. 6, a photograph specified by FGF2 and neovascularization (+ +)) were found to be significantly greater in tooth root elongation and periodontal bone formation than those of the negative control group and the other FGF2-experimental group, but the degrees of the elongation and formation were found to be less than those of the FGF18-experimental group (Example 1; in FIG. 6, a photograph specified by FGF18 and neovascularization (+ -)). Meanwhile, although the teeth of the FGF18-experimental group did not considerably exhibit neovascularization, they were considerably promoted in tooth root elongation and periodontal bone formation, as compared with the other experimental groups.

The above discussion indicates that the present tooth root formation promoting factor containing FGF18 as an active ingredient has excellent tooth root formation (elongation) activity and also, has an activity of promoting formation of periodontal tissues supporting the formed tooth root; e.g., periodontal ligament and alveolar bone. (Referential Example 1: Expression of endogenous FGF18 gene)

In Referential Example 1, a mammalian, at a stage when tooth root formation was initiated, was examined as to whether or not endogenous FGF18 expressed in the tooth roots (including dental pulps).

Incisors were extracted from ten-week-old mice, which are known to continue to form tooth roots. Under a stereomicroscope, root apex tissue and dental pulps of root-crown were separated from each incisor. The thus-obtained root apex tissue samples and dental pulp samples were stored at 4°C in the presence of RNAlater (product of QIAGEN) exhibiting RNase inhibitory activity.

Total RNA was extracted from each of the thus-obtained root-crown samples with QuickGene (product of Fuji Photo Film Co. Ltd.). Specifically, the root-crown sample (including hard tissue) was thoroughly pulverized with a disposable plastic pestle in the presence of a lysis buffer, followed by centrifugation. Thereafter, the supernatant was mixed with a predetermined amount of ethanol, and then the resultant mixture was applied to the QuickGene column. The extracted RNA was analyzed with a spectrophotometer for the concentration and 260/280 ratio. Subsequently, cDNA of the root-crown sample was prepared using RNA samples (300 ng) exhibiting a 260/280 ratio of 1.8 or higher. In this cDNA preparation, reverse transcription was carried out using SuperScriptII (product of Invitrogen Corp.). The FGF18 expression level of the sample was determined by quantitative polymerase chain reaction (qPCR). Specifically, the FGF18-specific primers were fabricated using design software on the Internet (see http://frodo.wi.mit.edu/cgi-bin/primer3/primer3_www.cgi); the quantitative PCR was carried out using Platinum SYBR Green qPCR SuperMix-UDG (product of Invitrogen Corp.) and DNA engine Opticon (product of Bio-Rad Laboratories, Inc.); and the FGF18 expression level was evaluated by comparing with that of β-actin serving as an internal standard substance.

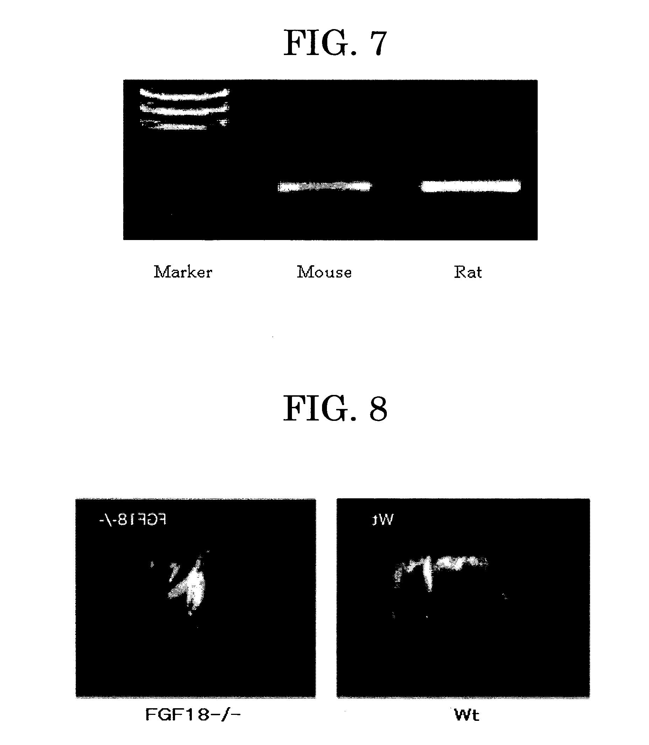

Similarly, six-week-old rats, at a stage when tooth root formation was initiated, were treated, to thereby evaluate endogenous FGF18 expression levels in dental pulps of root-crown. The results are shown in FIG. 7.

From the data obtained from the quantitative PCR, endogenous FGF18-gene expression was observed in tooth roots (including dental pulps) of mice and rats forming tooth roots (FIG. 7). Notably, the fact that FGF18 specifically expresses in elongating tooth roots (including dental pulps) of a mammalian has not conventionally known, and this fact has newly been found out by the present inventors.

### (Example 2: FGF8)

The procedure of Example 1 was repeated, except that FGF8 (product of PeproTech Co.) was used instead of FGF18, to thereby prepare FGF8-impregnated heparin acrylic beads of Example 2 (a preferred embodiment of the present tooth root formation promoting factor).

Similar to Example 1, the FGF8-impregnated heparin acrylic beads of Example 2 were embedded in the dental pulps of the embryonic teeth extirpated from wild-type mice, followed by transplant culturing. Also, the teeth obtained after transplant culturing and the periodontal bone were observed with soft X-ray photography. Separately, the above procedure was repeated, except that the embryonic teeth extirpated from the wild-type mice were substituted with embryonic teeth extirpated from FGF18-gene knockout embryonic mice (embryonic age: 18.5 days). The results are shown in FIG. 8.

### [Results (Example 2)]

The teeth of the experimental group in which FGF8 was applied to the embryonic teeth extirpated from the wild-type mice were found to exhibit tooth root elongation (Example 2; in FIG. 8, a photograph specified by Wt), indicating that FGF8 has tooth root elongation promotion activity similar to FGF18. Also, the teeth of the experimental group in which FGF8 was applied to the embryonic teeth extirpated from the FGF18-gene knockout mice were found to exhibit tooth root elongation (in FIG. 8, a photograph specified by FGF18 -/-), indicating that FGF8 has an activity of replacing physiological functions of FGF18.

The data obtained from Example 1 and Comparative Examples 1 and 2 indicate that the present tooth root formation promoting factor containing FGF18 as an active ingredient very effectively promotes formation of tooth roots and periodontal tissues supporting them; e.g., periodontal ligament and alveolar bone.

Also, from the data obtained from Referential Example 1, FGF18 is first elucidated to be a important factor involved in tooth root formation of a mammalian at a stage when tooth root formation is initiated, indicating that the present tooth root formation promoting factor containing FGF18 as an active ingredient has excellent tooth root formation promoting activity.

Further, from the data obtained from Example 2, FGF8 has the same tooth root formation promoting activity as FGF18, suggesting that effectiveness of FGF8 family proteins (FGF8, FGF17 and FGF18) in promoting formation of tooth roots and periodontal tissues supporting them; e.g., periodontal ligament and alveolar bone.

### Industrial Applicability

The present tooth root formation promoting factors and the present method for promotion of tooth root formation can be expected to be clinically applied to various aspects of dental therapy where promotion of tooth root formation is desired. Conceivably, the present promoting factors and method are particularly advantageously used as the followings (1) to (7):
(1) use thereof in pediatric dentistry can induce tooth root elongation in root-forming teeth, which have suffered from disorders caused by trauma or infection in the process of tooth root formation and being prevented from elongating;
(2) use thereof in orthodontic dentistry can induce regeneration of tooth roots resorbed due to orthodontic force accompanying orthodontic therapy;
(3) use thereof in general dentistry can induce regeneration of tooth roots resorbed caused by lesions in root apices (e.g., radicular cyst);
(4) use thereof in general dentistry can induce regeneration of tooth root-periodontal tissue after intentional reimplantation for the treatment of root fracture, etc.;
(5) use thereof in oral surgery can promote formation of tooth root-periodontal tissue of autotransplanted supernumerary teeth (third molars (i.e., wisdom teeth));
(6) use thereof in regenerative medicine can be expected to induce regeneration of tooth root-periodontal tissue after autotransplantation of juvenile teeth which have been stored in a frozen state; and
(7) use thereof in general dentistry can be expected to induce regeneration of cementum, periodontal ligament and alveolar bone resorbed due to, for example, periodontal disease.

## Claims

1. A tooth root formation promoting factor comprising, a protein belonging to the FGF8 subfamily as an active ingredient.

2. A method for promotion of tooth root formation comprising, administering a protein belonging to the FGF8 subfamily to a mammalian.
